# EUROPÄISCHE PATENTSCHRIFT

(11) **EP 3 106 191 B1**
(45) Veröffentlichungstag und Bekanntmachung des Hinweises auf die Patenterteilung: **13.03.2019**
(21) Anmeldenummer: 15172165.1
(22) Anmeldetag: 15.06.2015
(51) Int. Cl.: A61M 5/31, A61M 5/32, A61M 5/34

(54) **SICHERHEITSVORRICHTUNG FÜR EINE SPRITZE**
SAFETY DEVICE FOR A SYRINGE
DISPOSITIF DE SÉCURITÉ POUR UNE SERINGUE

(43) Veröffentlichungstag der Anmeldung: 21.12.2016
(73) Patentinhaber: Gerresheimer Regensburg GmbH, 93047 Regensburg (DE)
(72) Erfinder: Fraas, Andreas, 92224 Amberg (DE); Vogl, Maximilian, 92708 Mantel (DE)
(74) Vertreter: Hannke, Christian

(56) Entgegenhaltungen:
- EP-A1- 1 797 857
- WO-A1-03/018092
- WO-A1-2014/131985
- WO-A2-03/047657
- US-A- 2 695 613
- US-A- 2 834 346
- US-A- 5 843 041
- US-A1- 2013 281 970

## Beschreibung

Die Erfindung betrifft eine Sicherheitsvorrichtung zur Vermeidung von Stichverletzungen für eine Spritze mit einem Spritzenkörper und einem an dem distalen Ende des Spritzenkörpers angeordnetem Stechmittel, umfassend ein sich entlang einer axialen Richtung (X) erstreckendes Hülsenelement, welches zumindest teilweise das Stechmittel und den Spritzenkörper umschließt, und ein Kragenelement, welches an dem distalen Ende des Spritzenkörpers angeordnet ist und die Sicherheitsvorrichtung in axialer Richtung (X) arretiert, wobei das Kragenelement zumindest einen Führungsvorsprung aufweist, welcher in zumindest einer Führungskulisse des Hülsenelements bei einer Relativbewegung des Spritzenkörpers zu der Sicherheitseinrichtung im Wesentlichen entlang der axialen Richtung geführt ist.

Gattungsgemäße Sicherheitsvorrichtungen zur Vermeidung von Stichverletzungen sind aus dem Stand der Technik bekannt. Insbesondere bei vorgefüllten Spritzen ist die Verwendung derartiger Sicherheitsvorrichtungen sinnvoll. Die Handhabung derartiger Spritzen ist sehr einfach, da das Medium nicht vor der Anwendung in die Spritze transferiert werden muss. Weiterhin ist, selbst im Notfall, die Wahrscheinlichkeit der Anwendung eines falschen Medikaments sehr gering. Für Impfstoffe und zahlreiche andere Medikamente sind sie heutzutage das Primärpackmittel erster Wahl. Diese Spritzen sind üblicherweise aus Glas oder Kunststoff (beispielsweise COC, COP) hergestellt und müssen mit Schutzkappen ausgestattet sein, um Beschädigungen und/oder eine Kontaminierung der Kanüle vor der Anwendung der Spritze zu vermeiden. Darüber hinaus ist es wichtig, nach Gebrauch der Spritze die Kanüle zu sichern, um Stichverletzungen zu vermeiden. Dabei kann ein unvorsichtiges Wiederaufsetzten der Schutzkappe auf die Kanüle Stichverletzungen verursachen. Oft ist die entsprechende Schutzkappe nicht mehr auffindbar oder es wird vergessen, diese wiederaufzusetzen, wodurch ein vermeidbares Verletzungsrisiko gegeben ist. Demzufolge wurden Nadelschutzeinrichtungen entwickelt, welche fest mit der Spritze verbunden sind und die Nadel nach Verwendung der Spritze automatisch wieder aufnehmen. Eine solche Nadelschutzeinrichtung gemäß dem Oberbegriff von Anspruch 1 beispielsweise in DE 11 2009 001 083 T5 offenbart. Hierbei wird eine federangetriebene Sicherheitshülse gezeigt, welche in einem ausgefahrenen Zustand die Kanüle umgibt und diese gegen Verletzungen der Anwender sichert. Die Sicherheitshülse weist dabei eine Kurvenbahn auf, in welcher mindestens ein Führungsstift läuft, wodurch verschiedene Positionen der Sicherheitshülse in Abhängigkeit zur Nadelspitze realisiert werden können.

Der mindestens eine Führungsstift muss dabei über einen Kragen an der Frontgeometrie der Spritze befestigt werden oder muss auf andere Weise fest mit der Spritze verbunden werden. Der Kragen mit dem Führungsstift darf, um Manipulation oder Fehlbenutzung vorzubeugen, nicht oder nur schwer von der Spritze mit einer Kanüle entfernbar sein. Demnach ist ein entsprechend fester Sitz in axialer Richtung notwendig.

Daraus ergibt sich das Problem, dass die Sicherheitshülse bei einem fest sitzenden Führungsstift aufgrund der Kurvenbahn eine gewisse Rotation um die Längsachse des Spritzenkörpers erfährt. Die resultierende Rotation der auf der Haut des Patienten befindlichen Sicherheitshülse wird vom Patienten als unangenehm empfunden, da die Rotation der Sicherheitshülse die Haut um die Einstichstelle herum verdreht.

Andererseits ist eine zuverlässige und gleichzeitig leichtgängige Rotation eines Kragens mit Führungsstift schwierig zu realisieren, da gerade der maßgebliche Frontbereich insbesondere bei Glasspritzen sehr ungenau geformt wird beziehungsweise unterschiedliche Ausgestaltungen der Frontgeometrie bei Kunststoffspritzen verschiedener Hersteller existieren.

US 2 834 346 A offenbart eine Spritze mit einem Nadelaufsatz. In einem ursprünglichen Zustand ist der mit dem zu verabreichenden Medium befüllte Spritzenkörper mit einer Membran abgeschlossen. Der Nadelaufsatz besteht aus einem Verbindungselement, an welchem die Nadel befestigt ist. Die Nadel ist weiterhin in einer Schutzkappe angeordnet, welche mit dem Verbindungselement verbunden ist. Das Endstück des Spritzenkörpers ist mit Vorsprüngen und das Verbindungselement ist mit nach innen abstehenden Zungen ausgestattet. Wird der Nadelaufsatz auf den Spritzenkörper aufgesetzt, durchbricht ein innerhalb des Verbindungselements liegender Abschnitt der Nadel die Membran und die Zungen gleiten über die Vorsprünge und greifen in diese ein. Durch den Eingriff sitzt der Nadelaufsatz nun fest auf dem Spritzenkörper. Durch diesen festen Sitz kann mit einem Ziehen an der Schutzkappe in distaler Richtung die Schutzkappe von dem Verbindungselement abgetrennt werden.

US 2 695 613 A zeigt einen Nadelaufsatz. Der versiegelte und der mit der Nadel zu versehende Spritzenkörper ist an seinem distalen Ende mit einem Vorsprung ausgestattet. Der Nadelaufsatz weist Laschen auf, welche beim Aufsetzen auf den Spritzenkörper in den Vorsprung eingreifen, sodass die Nadel fest mit dem Spritzenkörper verbunden ist.

WO 2014/131985 A1 beschreibt eine Nadelschutzvorrichtung für eine Spritze. Die Nadelschutzvorrichtung umfasst einen inneren Körper und einen äußeren Körper. Eine brechbare Verbindung für das Verbinden der Nadelschutzvorrichtung mit einem Kragenelement ist vorhanden. Ein Schutz der Nadel nach der Injektion wird durch Wiederaufstecken des Nadelschutzes realisiert werden.

WO 03 04 7657 A2 zeigt einen Adapter für Kartuschen. Der Adapter wird über einen Eingriff von laschenartigen Elementen an einem Vorsprung am distalen Ende der Kartusche gehalten.

EP 1 797 857 A1 beschreibt einen Adapter für ein Vial. Der Adapter umfasst eine Nadel, welche beim Aufsetzen durch das das Vial verschließende Siegel gestoßen wird. Der Adapter umfasst weiterhin einen weiblichen Luer-Anschluss, wodurch beispielsweise mit einer Spritze eine Verbindung hergestellt werden kann.

US 5 843 041 A offenbart eine Sicherheitshülse für eine Spritze. In der Sicherheitshülse ist ein Befestigungselement angeordnet. Dieses Befestigungselement ist innerhalb einer Führung verschiebbar. Die Spritze wird am proximalen Ende in die Hülse eingeführt. Durch die Einführbewegung in distaler Richtung wird das Befestigungselement zunächst bis zu einem Anschlag mitgeschoben. Nach dem Anschlag führt eine weitere Bewegung der Spritze in distaler Richtung dazu, dass diese an dem Befestigungselement einrastet. Die Relativbewegung erfolgt geradlinig. WO 03/01892 A1 zeigt eine Befestigungsvorrichtung für Injektionsnadeln zum Aufstecken auf ein Gewinde eines Injektionsgeräts. Entlang der Mantelfläche der Befestigungsvorrichtung sind Federelemente angeordnet, welche Nocken in das Gewinde drücken. Durch die Klemmkraft der Nocken wird die Befestigungsvorrichtung gehalten. Dabei ist eine Verwendung der Befestigungsvorrichtung mit unterschiedlichen Gewinden bzw. Injektionsgeräten möglich.

US 2013/281970 A1 offenbart eine Sicherheitsvorrichtung für eine Spritze. Die hülsenförmige Sicherheitsvorrichtung ist über ein Kragenelement, welches am distalen Ende der Spritze angeordnet ist mit der Spritze verbunden. Dabei ist die hülsenförmige Sicherheitsvorrichtung über eine Führung an dem Kragenelement geführt. Die Sicherheitsvorrichtung ist zunächst über dem Spritzenkörper angeordnet. Nach dem Injektionsvorgang wird die Sicherheitsvorrichtung über die Nadel geschoben. In dieser Position kann die Sicherheitsvorrichtung arretiert werden. Anschließend kann die Sicherheitsvorrichtung mit der Nadel von dem Spritzenkörper abgeknickt werden, sodass die beiden Teile getrennt entsorgt werden können. Es findet eine geradlinige Relativbewegung eines Hülsenelementes statt.

Aufgabe der vorliegenden Erfindung ist es, eine Sicherheitsvorrichtung zur Vermeidung von Stichverletzungen für eine Spritze zur Verfügung zu stellen, welche die eingangs genannten Probleme löst. Der Schutz vor Stichverletzungen vor und nach dem Gebrauch der Spritze soll vermieden werden. Weiterhin soll die Positionierung und Funktion der Sicherheitsvorrichtung nicht durch die Ausgestaltung der Frontgeometrie des Spritzenkörpers beeinträchtigt werden.

Diese Aufgabe wird gelöst durch eine Sicherheitsvorrichtung zur Vermeidung von Stichverletzungen für eine Spritze mit unter anderem einem Spritzenkörper und einem an dem distalen Ende des Spritzenkörpers angeordnetem Stechmittel, umfassend ein sich entlang einer axialen Richtung (X) erstreckendes Hülsenelement, welches zumindest teilweise das Stechmittel und den Spritzenkörper umschließt, und ein Kragenelement, welches an einem distalen Endbereich des Spritzenkörpers angeordnet ist und die Sicherheitsvorrichtung in axialer Richtung (X) arretiert, wobei das Kragenelement zumindest einen Führungsvorsprung aufweist, welcher in zumindest einer Führungskulisse des Hülsenelements bei einer Relativbewegung des Spritzenkörpers zu dem Hülsenelement im Wesentlichen entlang der axialen Richtung geführt ist. Die Sicherheitsvorrichtung zeichnet sich dadurch aus, dass das Kragenelement in einer Umfangsrichtung (U) rotierbar an dem distalen Endbereich des Spritzenkörpers angeordnet ist und zumindest ein Toleranzausgleichselement aufweist, welches mit dem Spritzenkörper mechanisch in Wirkkontakt ist, so dass Toleranzen des Spritzenkörpers ausgleichbar sind.

Bei einer Anwendung der Spritze wird die Spritze mit der Sicherheitseinrichtung gegen die Haut des Patienten gedrückt. Durch die Relativbewegung des Spritzenkörpers zu dem Hülsenelement und die Führung des Führungsvorsprungs in der Führungskulisse wird eine Rotation des Kragenelements entlang einer Umfangsrichtung (U) verursacht. Das Hülsenelement schiebt sich dadurch vorzugsweise über den Spritzenkörper, wodurch das Stechmittel, welches eine Kanüle, eine Nadel oder auch eine Lanzette sein kann, durch eine entsprechende Öffnung in dem Hülsenelement hindurchtritt. Somit wird eine Rotation des Hülsenelements auf der Haut des Patienten um die Einstichstelle herum vermieden.

Weiterhin werden durch das zumindest eine Toleranzausgleichselement auftretende Toleranzen des Spritzenkörpers an seinem distalen Endbereich, welcher auch als Spritzenfrontgeometrie bezeichnet werden kann, ausgeglichen. Unter Toleranzen ist hierbei eine Abweichung der Form bzw. der Abmessungen des Spritzenkörpers von der idealen Form bzw. den idealen Abmessungen in diesem distalen Endbereich zu verstehen. Derartige Toleranzen können bei Glasspritzen bei der Herstellung auftreten. Ebenso können solche Toleranzen durch abweichende Formen der Spritzenfrontgeometrie bei Spritzen unterschiedlicher Hersteller auftreten. Derartige Toleranzen können beispielsweise den Durchmesser der Spritzenfrontgeometrie, die Länge der Spritzenfrontgeometrie oder Unregelmäßigkeiten bei der Rundung der Spritzenfrontgeometrie umfassen. Um eine optimale leichtgängige Führung des Führungsvorsprungs in der Führungskulisse zu gewährleisten, ist es notwendig, dass das Kragenelement sich an einer idealen Montageposition befindet. Geringste Abweichungen von dieser Position können bereits die Führung beeinträchtigen. Durch das zumindest eine Toleranzausgleichselement, welches mit dem Spritzenkörper mechanisch in Wirkkontakt steht, sind nun derartige Toleranzen des Spritzenkörpers ausgleichbar. Dadurch kann eine genaue Positionierung des Kragenelements auf dem Spritzenkörper erreicht werden, wodurch wiederum eine optimale Führung des Führungsvorsprungs in der Führungskulisse erreicht wird. Weiterhin ist eine Verwendung der Sicherheitseinrichtung für unterschiedliche Ausgestaltungen der Frontgeometrie bei Kunststoffspritzen verschiedener Hersteller gewährleistet.

Gemäß einer besonders bevorzugten Ausführungsform ist das Kragenelement im Wesentlichen als hohler Kreiszylinder ausgebildet. Bevorzugt weist der Kreiszylinder eine Mantelfläche auf, an der der zumindest eine Führungsvorsprung angeordnet ist. Vorzugsweise erstreckt sich der zumindest ein Führungsvorsprung radial von der Mantelfläche weg. Weiterhin bevorzugt ist der Führungsvorsprung als Kreiszylinder beziehungsweise als Stift ausgebildet. Vorteilhafterweise sind an der Mantelfläche zwei sich diametral gegenüberliegende Führungsvorsprünge angeordnet. Demzufolge würde auch das Hülsenelement zwei sich diametral gegenüberliegende Führungskulissen aufweisen, in denen jeweils ein Führungsvorsprung geführt wird.

Vorzugsweise ist der Spritzenkörper als hohler Kreiszylinder ausgestaltet und weist in seinem distalen Endbereich ein konisches Endstück auf, an welchem das Stechmittel angeordnet ist. Bevorzugt ist an dem konischen Endstück ein Vorsprung ausgebildet, an welchem eine Stirnfläche des distalen Endes des Kragenelements eingreifbar ist, wodurch das Kragenelement und somit die Sicherheitsvorrichtung in axialer Richtung arretierbar sind.

Nach einem besonders bevorzugten Gedanken der Erfindung ragt das zumindest eine Toleranzausgleichselement entlang der axialen Richtung (X) über ein distales Ende des Kragenelements hinaus. Durch eine derartige Ausgestaltung wird eine ideale Positionierung des Kragenelements in axialer Richtung gewährleistet.

Nach einem weiteren besonders bevorzugten Gedanken der Erfindung erstreckt sich das zumindest eine Toleranzausgleichselement ausgehend von dem Kragenelement entlang einer radialen Richtung (R) nach innen zu dem Spritzenkörper hin. Durch eine derartige Ausgestaltung wird eine ideale Positionierung des Kragenelements in radialer Richtung gewährleistet. Bevorzugt ist das zumindest eine Toleranzausgleichselement stempelartig ausgebildet. Vorzugsweise weist das stempelartige Toleranzausgleichselement dabei eine Anliegefläche auf, welche mit dem Spritzenkörper in Wirkkontakt bringbar ist. Diese Anliegefläche weist bevorzugt geringe Abmessungen bevorzugt zwischen 0,5 mm² und 5 mm² und besonders bevorzugt zwischen 0,5 mm² und 1,5 mm² auf, so dass die Reibung zwischen dem Kragenelement und dem Spritzenkörper bei der Rotation des Kragenelements reduziert ist.

Bevorzugt ist das zumindest eine Toleranzausgleichselement elastisch ausgestaltet. Die eventuell auftretenden Toleranzen des Spritzenkörpers können somit durch Deformation des zumindest einen Toleranzausgleichselements ausgeglichen werden.

Gemäß einer besonders bevorzugten Ausführungsform umfasst das Kragenelement drei Toleranzausgleichselemente. Vorzugsweise sind die Toleranzausgleichselemente an dem distalen Ende des Kragenelements derart angeordnet, dass jeweils zwei Mittelachsen der Toleranzausgleichselemente einen Winkel (α, β, γ) einschließen. Bevorzugt betragen die Winkel α = β = γ = 120°. Es ist jedoch auch denkbar, dass unterschiedliche Winkel von den entsprechenden Mittelachsen eingeschlossen werden. Durch eine derartige Ausgestaltung kann insbesondere eine verkippte Position des Kragenelements an dem Spritzenkörper vermieden werden.

Vorzugsweise ist das zumindest eine Toleranzausgleichselement integral mit dem Kragenelement ausgebildet. Ein derartiges Kragenelement ist besonderes einfach und kostengünstig herzustellen. Es ist aber auch denkbar, dass das Kragenelement in seinem Wandbereich zumindest eine Ausnehmung aufweist, in der das zumindest eine Toleranzausgleichselement anordenbar ist. Eine solche Ausgestaltung ermöglicht es, das zumindest eine Toleranzausgleichselement und das Kragenelement aus unterschiedlichen Materialien herzustellen.

Gemäß der Erfindung umfasst das Kragenelement einen distalen Bereich, in welchem die Wandung des Kragenelements zumindest zwei Schlitze aufweist, welche sich in axialer Richtung (X) erstrecken. Durch derartige Schlitze ist eine Anpassung des Kragenelements an unterschiedliche Spritzenkörperformen beziehungsweise Spritzenkörperdurchmesser gewährleistet. Ferner ist durch die Schlitze das Aufbringen des Kragenelements auf den Spritzenkörper erleichtert. Beim Aufbringen des Kragenelements auf den Spritzenkörper wird üblicherweise das Kragenelement auf den Spritzenkörper geschoben. Weist nun der Spritzenkörper einen Vorsprung oder ein Verdickung auf, welche beispielsweise zur Arretierung des Kragenelements in axialer Richtung dienen können, kann ein Aufbringen erschwert werden. Durch die vorteilhaften Schlitze ist jedoch eine geringe Aufweitung des Kragenelements möglich, wodurch dieses leichter auf den Spritzenkörper aufgeschoben werden kann.

Vorzugsweise umfasst das Kragenelement einen proximalen Bereich, welcher einen Innenkonus aufweist. Durch diesen proximalen Bereich wird ein zweiter Lagerpunkt für das Kragenelement an dem Spritzenkörper im Bereich der Spritzenschultern realisiert.

Bevorzugt ist zumindest in dem proximalen Bereich des Kragenelements zumindest ein in radialer Richtung hervorragendes Element angeordnet. Vorteilhafterweise erstreckt sich das hervorragende Element in radialer Richtung nach innen, ausgehend von dem Kragenelement zu dem Spritzenkörper hin. Durch dieses zumindest eine hervorragende Element wird die Auflagefläche des Kragenelements auf dem Spritzenkörper reduziert, wodurch auch die Reibung zwischen dem Kragenelement und dem Spritzenkörper bei einer Rotation des Kragenelements reduziert wird. Besonders bevorzugt sind drei hervorragende Elemente in dem proximalen Bereich des Kragenelements angeordnet, wodurch ein optimale Lagerung des Kragenelements an dem Spritzenkörper gewährleistet wird.

Vorzugsweise weist die Sicherheitsvorrichtung mindestens ein Federelement auf, das mit dem Spritzenkörper wirkverbunden ist und der Relativbewegung des Spritzenkörpers zu der Sicherheitsvorrichtung entgegenwirkt. Demnach bleibt die Kanüle bis zur vorgesehenen Anwendung innerhalb des Hülsenelements. Bei der Anwendung muss das Hülsenelement gegen die Federkraft verschoben werden, damit die Kanüle durch die Öffnung des Hülsenelements hindurchtreten kann. Nach Gebrauch der Spritze schiebt sich automatisch, angetrieben durch die Federkraft des Federelements, das Hülsenelement wieder über die Kanüle. Durch die Führung des Führungsvorsprungs in der Führungskulisse rotiert das Kragenelement entgegen der Umfangsrichtung (U). Der Anwender ist somit vor Stichverletzungen mit der gebrauchten kontaminierten Kanüle geschützt. Bevorzugt umfasst das Federelement eine Spiralfeder. Denkbar sind aber auch anderweitige Federarten, wie beispielsweise Schenkelfedern oder Torsionsfedern. Ferner wäre vorstellbar, das Federelement als ein Elastomer auszubilden.

Bevorzugt bestehen das Kragenelement und/oder das zumindest eine Toleranzausgleichselement aus gleitmodifiziertem Polyoxymethylen (POM) oder aus einer Silikonverbindung, wodurch weiterhin die Reibung zwischen Kragenelement und Spritzenkörper bei einer Rotation des Kragenelements reduziert werden kann.

Vorzugsweise weisen das Kragenelement und/oder das zumindest eine Toleranzausgleichselement eine gleitfördernde Beschichtung auf.

Weitere Vorteile, Ziele und Eigenschaften der vorliegenden Erfindung werden anhand nachfolgender Beschreibung der anliegenden Figuren erläutert. Gleichartige Komponenten können in den verschiedenen Ausführungsformen gleiche Bezugszeichen aufweisen.

In den Figuren zeigen:
- Fig.1: einen Abschnitt einer Spritze ohne Sicherheitsvorrichtung;
- Fig.2: eine Schnittdarstellung einer Spritze mit Sicherheitsvorrichtung;
- Fig.3: eine Schnittdarstellung einer Spritze mit Sicherheitsvorrichtung;
- Fig.4: eine isometrische Ansicht eines Kragenelements;
- Fig.5: eine isometrische Ansicht eines Kragenelements;
- Fig.6: eine Draufsicht eines Kragenelements;
- Fig.7: eine Schnittdarstellung eines Kragenelements;
- Fig.8: eine Rückansicht eines Kragenelements;
- Fig.9: eine Schnittdarstellung eines Kragenelements.

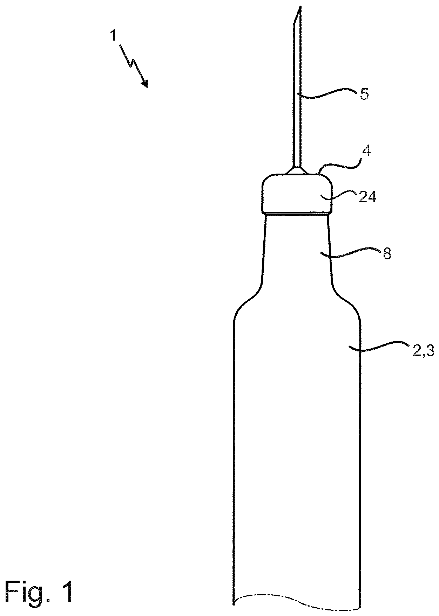

In Fig. 1 ist ein Abschnitt einer Spritze (2) ohne Sicherheitsvorrichtung (1) dargestellt. Die Spritze umfasst einen Spritzenkörper (3), welcher als hohler Kreiszylinder ausgestaltet ist. Der Spritzenkörper weist einen distalen Endbereich (8) mit einem distalen Ende (4) auf. An dem distalen Ende (4) ist ein Stechmittel (5) angeordnet. Dieses Stechmittel (5) ist über eine Bohrung in dem distalen Endbereich (8) mit dem Hohlraum des Spritzenkörpers (3) verbunden, so dass das zu injizierende Medium bei einer Anwendung der Spritze (2) durch das Stechmittel (5) treten kann. Der distale Endbereich (8) ist als konisches Endstück ausgestaltet, welches einen kleineren Außendurchmesser als der Spritzenkörper (3) aufweist. Weiterhin weist die Spritze einen Übergangsbereich (25) auf, in dem der Außendurchmesser des Spritzenkörpers in den Außendurchmesser des Endstücks übergeht. Weiterhin ist an dem distalen Endbereich ein Vorsprung (24) angeordnet.

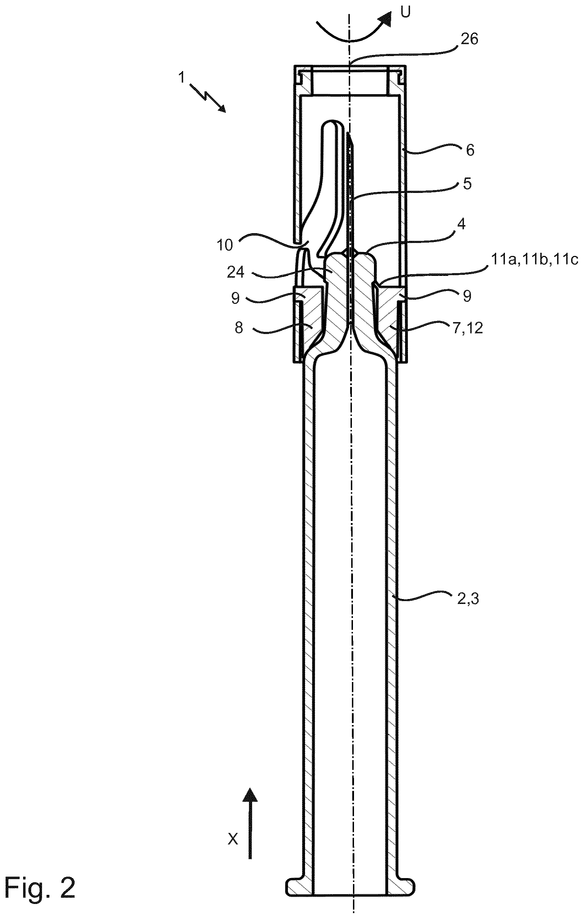

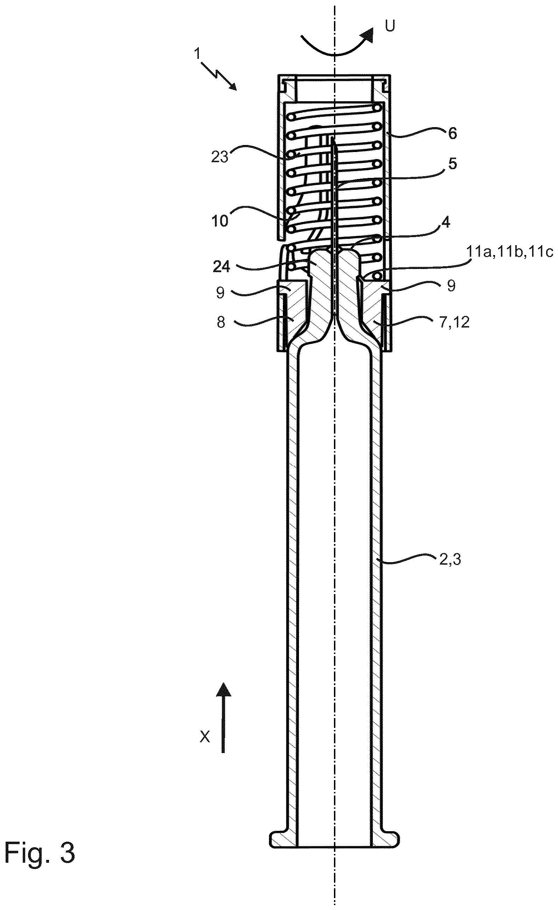

In Fig. 2 und in Fig. 3 ist eine Spritze mit einer Sicherheitsvorrichtung (1) zur Vermeidung von Stichverletzungen gezeigt. Die Spritze (2) umfasst einen Spritzenkörper (3) und ein an dem distalen Ende (4) des Spritzenkörpers (3) angeordnetes Stechmittel (5). Die Sicherheitsvorrichtung (1) umfasst ein sich entlang einer axialen Richtung (X) erstreckendes Hülsenelement (6), welches zumindest teilweise das Stechmittel (5) und den Spritzenkörper (3) umschließt, und ein Kragenelement (7), welches an einem distalen Endbereich (8) des Spritzenkörpers (3) angeordnet ist und die Sicherheitsvorrichtung (1) in axialer Richtung (X) arretiert. Die Arretierung in axialer Richtung (X) wird durch einen Vorsprung (24) bzw. eine Verdickung an dem distalen Ende (4) des Spritzenkörpers ermöglicht, an welchem das Kragenelement mit seinem distalen Ende anliegt.

Das Kragenelement (7) weist zwei Führungsvorsprünge (9) auf, welche in jeweils einer Führungskulisse (10) des Hülsenelements (6) bei einer Relativbewegung des Spritzenkörpers (3) zu dem Hülsenelement (6) im Wesentlichen entlang der axialen Richtung (X) geführt sind. Das Kragenelement (7) ist in einer Umfangsrichtung (U) rotierbar an dem distalen Endbereich (8) des Spritzenkörpers (3) angeordnet und weist zumindest ein Toleranzausgleichselement (11a, 11b, 11c) auf, welches mit dem Spritzenkörper (3) mechanisch in Wirkkontakt ist, so dass Toleranzen des Spritzenkörpers (3) ausgleichbar sind.

In Fig. 3 ist weiterhin eine Sicherheitsvorrichtung (1) dargestellt, welche ein Federelement (23) in Form einer Spiralfeder aufweist, das mit dem Spritzenkörper (3) wirkverbunden ist und der Relativbewegung des Hülsenelements (6) zu der Sicherheitsvorrichtung (1) entgegenwirkt. Demnach bleibt das Stechmittel (5) bis zur vorgesehenen Anwendung innerhalb des Hülsenelements (6). Bei der Anwendung muss das Hülsenelement (6) gegen die Federkraft verschoben werden, damit das Stechmittel (5) durch eine Öffnung (26) des Hülsenelements (6) hindurchtreten kann. Nach Gebrauch der Spritze (2) schiebt sich automatisch, angetrieben durch die Federkraft des Federelements (23), das Hülsenelement (6) wieder über das Stechmittel (5). Durch die Führung der Führungsvorsprünge (9) in den Führungskulissen (10) rotiert das Kragenelement (7) entgegen der Umfangsrichtung (U). Der Anwender ist somit vor Stichverletzungen mit dem gebrauchten und kontaminierten Stechmittel geschützt.

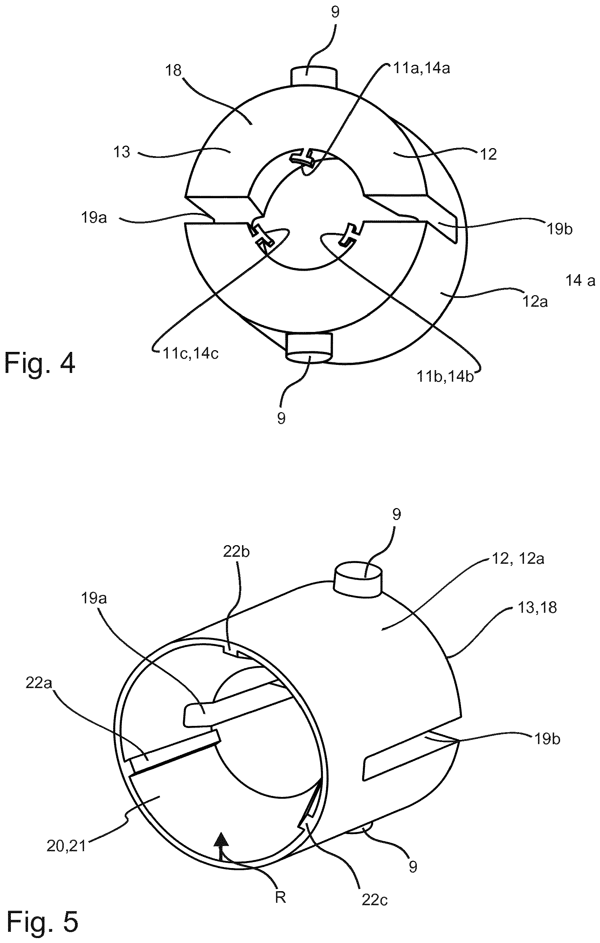

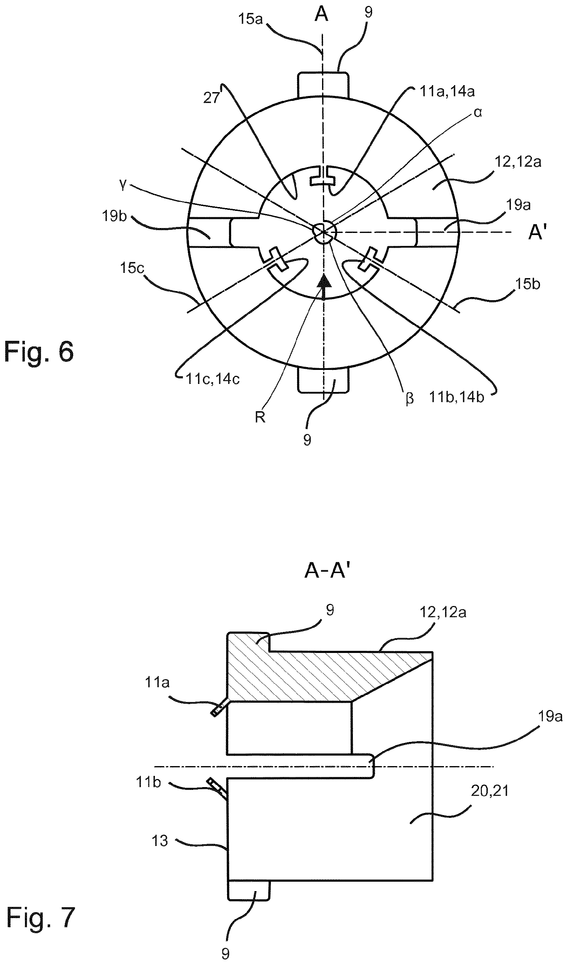

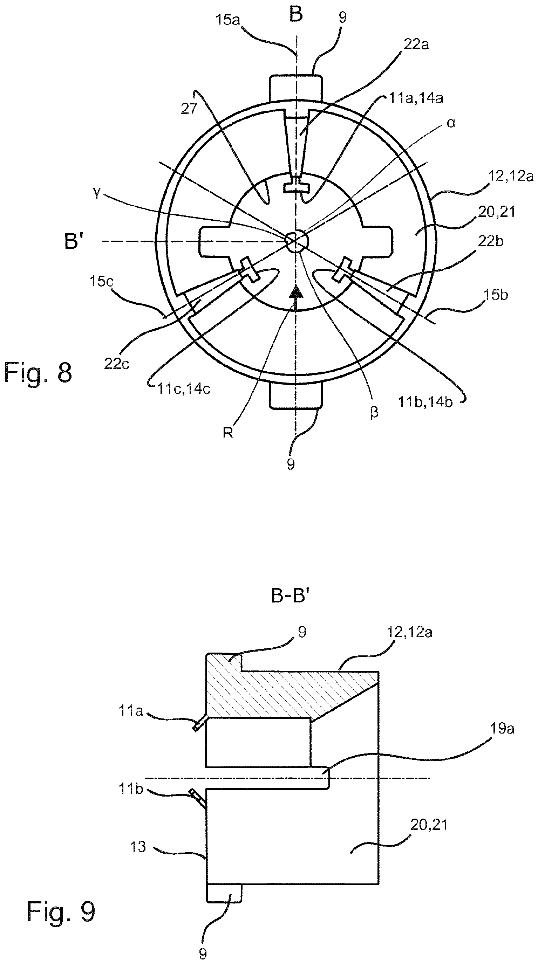

In Fig. 4 und Fig. 5 ist jeweils eine isometrische Ansicht des Kragenelements (7) gezeigt, wobei in Fig. 4 das distale Ende des Kragenelements (7) und in Fig. 5 das proximale Ende des Kragenelements (7) dargestellt ist. Ferner ist in Fig. 6 eine Draufsicht auf das distale Ende des Kragenelements (7) gezeigt und in Fig. 8 eine Draufsicht auf das proximale Ende des Kragenelements (7). Die Figuren 7 und 9 zeigen eine Schnittdarstellung entlang der Achsen A-A' (in Fig. 6) beziehungsweise B-B'(in Fig. 8).

Das Kragenelement (7) ist im Wesentlichen als hohler Kreiszylinder (12) ausgebildet. Der Kreiszylinder (12) weist eine Mantelfläche (12a) auf, an der zwei Führungsvorsprünge (9) angeordnet sind. Die Führungsvorsprünge erstrecken sich radial von der Mantelfläche (12a) nach außen weg und sind diametral gegenüberliegend angeordnet. Weiterhin sind diese als Kreiszylinder beziehungsweise als Stift ausgebildet.

Das Kragenelement (7) umfasst einen proximalen Bereich (20), welcher einen Innenkonus (21) aufweist. Dies ist in Fig. 5 ersichtlich. In diesem proximalen Bereich (20) des Kragenelements (7) sind drei hervorragende Elemente (22a, 22b, 22c) angeordnet, welche sich ausgehend von dem Kragenelement (7) in radialer Richtung (R) nach innen erstrecken. Die drei hervorragenden Elemente (22a, 22b, 22c) liegen in dem Übergangsbereich (25) des Spritzenkörpers an diesem an, wodurch ein weiterer Lagerpunkt des Kragenelements (7) an dem Spritzenkörper (3) realisiert wird. Dies ist in den Fig. 5 und 8 ersichtlich. Demzufolge liegt lediglich eine kleine Fläche an dem Spritzenkörper (3) an, wodurch bei einer Rotation des Kragenelements (7) die Reibung zwischen dem Kragenelement (7) und dem Spritzenkörper (3) reduziert ist.

Das Kragenelement (7) umfasst weiterhin einen distalen Bereich (18), in welchem die Wandung des Kragenelements (7) zwei Schlitze (19a, 19b) aufweist, welche sich in axialer Richtung (X) erstrecken. Diese Schlitze erstrecken sich weiterhin abschnittsweise in den proximalen Bereich (20) des Kragenelements (7), welcher den Innenkonus (21) aufweist. Durch die Schlitze (9) wird eine bessere Anpassung des Kragenelements (7) an unterschiedliche Spritzenkörper (3) beziehungsweise ein leichteres Aufbringen des Kragenelements (7) auf den Spritzenkörper (3) ermöglicht. Üblicherweise wird das Kragenelement (7) über das distale Ende (4) des Spritzenkörpers geschoben. Dieses distale Ende (4) weist einen Vorsprung (24) bzw. eine Verdickung auf. Durch die Schlitze (9) kann das Kragenelement (7) während des Aufbringens aufgeweitet werden, wodurch dieses leichter über den Vorsprung (24) geschoben werden kann.

Ferner weist das Kragenelement (7) drei Toleranzausgleichselemente (11a, 11b, 11c) auf. Die Toleranzausgleichselemente (11a, 11b, 11c) sind an dem distalen Ende (13) des Kragenelements (7) derart angeordnet, dass deren Mittelachsen (15a, 15b, 15c) bezüglich einander jeweils einen Winkel α = β = γ = 120° einschließen. Dies ist in Fig. 6 und Fig. 8 zu erkennen, welche eine Draufsicht des distalen Endes des Kragenelements (7) zeigen. In Fig. 7 ist weiterhin ein Schnitt entlang der Achse A-A' aus Fig. 6 gezeigt. Schließlich zeigt Fig. 9 einen Schnitt entlang der Achse B-B' aus Fig. 8. Fernern sind die Toleranzausgleichselemente (11a, 11b, 11c) integral mit dem Kragenelement (7) ausgebildet.

Die Toleranzausgleichselemente (11a, 11b, 11c) erstrecken sich ausgehend von dem Kragenelement (7) entlang einer radialen Richtung (R) nach innen und ragen somit in den Hohlraum des Kreiszylinders (12) hinein, wodurch eine ideale Positionierung des Kragenelements (7) in radialer Richtung (R) gewährleistet ist.

Ferner sind die Toleranzausgleichselemente (11a, 11b, 11c) stempelartig ausgebildet. Die stempelartigen Toleranzausgleichselemente (11a, 11b, 11c) weisen eine Anliegefläche (12a, 12b, 12c) auf, welche mit dem Spritzenkörper (3) in Wirkkontakt bringbar ist. Diese Anliegeflächen (12a, 12b, 12c) erstrecken sich entlang einer Umfangsrichtung des Innenkreises (27) des hohlzylindrischen Kragenelements (7).

Das zumindest eine Toleranzausgleichselement (11a, 11b, 11c) ragt entlang der axialen Richtung (X) über ein distales Ende (13) des Kragenelements (7) hinaus, wodurch eine ideale Positionierung des Kragenelements in axialer Richtung gewährleistet wird. Dies ist in Fig. 7 beziehungsweise Fig. 9 zu erkennen.

### Bezugszeichenliste

- 1: Sicherheitsvorrichtung
- 2: Spritze
- 3: Spritzenkörper
- 4: distales Ende des Spritzenkörpers
- 5: Stechmittel
- 6: Hülsenelement
- 7: Kragenelement
- 8: distaler Endbereich des Spritzenkörpers
- 9: Führungsvorsprung
- 10: Führungskulisse
- 11a, 11b,: Toleranzausgleichselement
- 11c: Toleranzausgleichselement
- 12: hohler Kreiszylinder
- 12a: Mantelfläche des Kreiszylinders
- 13: distales Ende des Kragenelements
- 14a, 14b: Anliegefläche
- 14c: Anliegefläche
- 15a, 15b,: Mittelachsen derToleranzausgleichselemente
- 15c: Mittelachse derToleranzausgleichselemente
- 18: distaler Bereich des Kragenelements
- 19a, 19b: Schlitze
- 20: proximaler Bereich des Kragenelements
- 21: Innenkonus
- 22a, 22b: hervorragendes Element
- 22c: hervorragendes Element
- 23: Federelement
- 24: Vorsprung
- 25: Übergangsbereich
- 26: Öffnung des Hülsenelements
- 27: Innenkreis des hohlzylindrischen Kragenelements
- X: axiale Richtung
- U: Umfangsrichtung
- R: radiale Richtung
- α, β, γ: Winkel

## Patentansprüche

1. Sicherheitsvorrichtung (1) zur Vermeidung von Stichverletzungen für eine Spritze (2) mit einem Spritzenkörper (3) und einem an dem distalen Ende (4) des Spritzenkörpers (3) angeordnetem Stechmittel (5), umfassend ein sich entlang einer axialen Richtung (X) erstreckendes Hülsenelement (6), welches zumindest teilweise das Stechmittel (5) und den Spritzenkörper (3) umschließt, und ein Kragenelement (7), welches an einem distalen Endbereich (8) des Spritzenkörpers (3) angeordnet ist und die Sicherheitsvorrichtung (1) in axialer Richtung (X) arretiert, wobei das Kragenelement (7) zumindest einen Führungsvorsprung (9) aufweist, welcher in zumindest einer Führungskulisse (10) des Hülsenelements (6) bei einer Relativbewegung des Spritzenkörpers (3) zu dem Hülsenelement (6) im Wesentlichen entlang der axialen Richtung (X) geführt ist,
wobei
das Kragenelement (7) in einer Umfangsrichtung (U) rotierbar an dem distalen Endbereich (8) des Spritzenkörpers (3) angeordnet ist und zumindest ein Toleranzausgleichselement (11a, 11b, 11c) aufweist, welches mit dem Spritzenkörper (3) mechanisch in Wirkkontakt ist, so dass Toleranzen des Spritzenkörpers (3) ausgleichbar sind, wobei die Relativbewegung des Spritzenkörpers (3) zu dem Hülsenelement (6) durch die Führung des Führungsvorsprungs (9) in der zumindest einen Führungskulisse (10) des Hülsenelements (6) die Rotation des Kragenelements (7) in der Umfangsrichtung (U) verursacht,
**dadurch gekennzeichnet, dass**
das Kragenelement (7) einen distalen Bereich (18) umfasst, in welchem die Wandung des Kragenelements (7) zumindest zwei Schlitze (19a, 19b) aufweist, welche sich in axialer Richtung (X) erstrecken.

2. Sicherheitsvorrichtung (1) nach Anspruch 1,
**dadurch gekennzeichnet, dass**
das Kragenelement (7) im Wesentlichen als hohler Kreiszylinder (12) ausgebildet ist, wobei der Kreiszylinder (12) eine Mantelfläche (12a) aufweist, an der der zumindest eine Führungsvorsprung (9) angeordnet ist.

3. Sicherheitsvorrichtung (1) nach Anspruch 1 oder 2,
**dadurch gekennzeichnet, dass**
das zumindest eine Toleranzausgleichselement (11a, 11b, 11c) entlang der axialen Richtung (X) über ein distales Ende (13) des Kragenelements (7) hinausragt.

4. Sicherheitsvorrichtung (1) nach einem der vorhergehenden Ansprüche,
**dadurch gekennzeichnet, dass**
das zumindest eine Toleranzausgleichselement (11a, 11b, 11c) sich ausgehend von dem Kragenelement (7) entlang einer radialen Richtung (R) nach innen erstreckt.

5. Sicherheitsvorrichtung (1) nach einem der vorhergehenden Ansprüche,
**dadurch gekennzeichnet, dass**
das zumindest eine Toleranzausgleichselement (11a, 11b, 11c) stempelartig ausgebildet ist, wobei das stempelartige Toleranzausgleichselement (11a, 11b, 11c) eine Anliegefläche (12a, 12b, 12c) aufweist, welche mit dem Spritzenkörper (3) in Wirkkontakt bringbar ist.

6. Sicherheitsvorrichtung (1) nach einem der vorhergehenden Ansprüche,
**dadurch gekennzeichnet, dass**
das zumindest eine Toleranzausgleichselement (11a, 11b, 11c) elastisch ausgestaltet ist.

7. Sicherheitsvorrichtung (1) nach einem der vorhergehenden Ansprüche,
**dadurch gekennzeichnet, dass**
das Kragenelement (7) drei Toleranzausgleichselemente (11a, 11b, 11c) umfasst, wobei Toleranzausgleichselemente (11a, 11b, 11c) an dem distalen Ende (13) des Kragenelements (7) derart angeordnet sind, dass jeweils zwei Mittelachsen (15a, 15b, 15c) der Toleranzausgleichselemente (11a, 11b, 11c) einen Winkel (α, β, γ) einschließen.

8. Sicherheitsvorrichtung (1) nach einem der vorhergehenden Ansprüche,
**dadurch gekennzeichnet, dass**
das zumindest eine Toleranzausgleichselement (11a, 11b, 11c) integral mit dem Kragenelement (7) ausgebildet ist.

9. Sicherheitsvorrichtung (1) nach einem der vorhergehenden Ansprüche,
**dadurch gekennzeichnet, dass**
das Kragenelement (7) einen proximalen Bereich (20) umfasst, welcher einen Innenkonus (21) aufweist.

10. Sicherheitsvorrichtung (1) nach Anspruch 9,
**dadurch gekennzeichnet, dass**
zumindest in dem proximalen Bereich (20) des Kragenelements (7) zumindest ein hervorragendes Element (22a, 22b, 22c) angeordnet ist, wobei das hervorragende Element (22a, 22b, 22c) sich ausgehend von dem Kragenelement (7) in radialer Richtung (R) nach innen erstreckt.

11. Sicherheitsvorrichtung (1) nach einem der vorhergehenden Ansprüche,
**dadurch gekennzeichnet, dass**
die Sicherheitsvorrichtung (1) mindestens ein Federelement (23) aufweist, das mit dem Spritzenkörper (3) wirkverbunden ist und der Relativbewegung des Hülsenelements (6) zu der Sicherheitsvorrichtung (1) entgegenwirkt.

12. Sicherheitsvorrichtung (1) nach einem der vorhergehenden Ansprüche,
**dadurch gekennzeichnet, dass**
das Kragenelement (7) und/oder das zumindest eine Toleranzausgleichselement (11a, 11b, 11c) aus gleitmodifiziertem Polyoxymethylen (POM) oder aus einer Silikonverbindung bestehen.

13. Sicherheitsvorrichtung (1) nach einem der vorhergehenden Ansprüche,
**dadurch gekennzeichnet, dass**
das Kragenelement (7) und/oder das zumindest eine Toleranzausgleichselement (11a, 11b, 11c) eine gleitfördernde Beschichtung aufweisen.

## Claims

1. Safety device (1) for preventing puncture wounds for a syringe (2) having a syringe body (3) and a piercing means (5) arranged at the distal end (4) of the syringe body (3), comprising a sleeve element (6) that extends along an axial direction (X) and encloses the piercing means (5) and the syringe body (3) at least in part, and a collar element (7) that is arranged on a distal end region (8) of the syringe body (3) and locks the safety device (1) in the axial direction (X), the collar element (7) having at least one guide projection (9) that is guided in at least one guide track (10) of the sleeve element (6) substantially along the axial direction (X) when the syringe body (3) is moved relative to the sleeve element (6),
the collar element (7) being arranged on the distal end region (8) of the syringe body (3) so as to be rotatable in a circumferential direction (U) and having at least one tolerance compensation element (11a, 11b, 11c) which is mechanically in operative contact with the syringe body (3) such that tolerances of the syringe body (3) can be compensated, the movement of the syringe body (3) relative to the sleeve element (6) causing the collar element (7) to rotate in the circumferential direction (U) by means of the guide projection (9) being guided in the at least one guide track (10) of the sleeve element (6),
**characterised in that**
the collar element (7) comprises a distal region (18) in which the wall of the collar element (7) has at least two slots (19a, 19b) that extend in the axial direction (X).

2. Safety device (1) according to claim 1,
**characterised in that**
the collar element (7) is formed substantially as a hollow circular cylinder (12), the circular cylinder (12) having a lateral surface (12a) on which the at least one guide projection (9) is arranged.

3. Safety device (1) according to either claim 1 or claim 2,
**characterised in that**
the at least one tolerance compensation element (11a, 11b, 11c) protrudes beyond a distal end (13) of the collar element (7) along the axial direction (X).

4. Safety device (1) according to any of the preceding claims,
**characterised in that**
the at least one tolerance compensation element (11a, 11b, 11c) extends inwards along a radial direction (R) from the collar element (7).

5. Safety device (1) according to any of the preceding claims,
**characterised in that**
the at least one tolerance compensation element (11a, 11b, 11c) is designed to be stamp-like, the stamp-like tolerance compensation element (11a, 11b, 11c) having a contact surface (12a, 12b, 12c) which can be brought into operative contact with the syringe body (3).

6. Safety device (1) according to any of the preceding claims,
**characterised in that**
the at least one tolerance compensation element (11a, 11b, 11c) is designed to be resilient.

7. Safety device (1) according to any of the preceding claims,
**characterised in that**
the collar element (7) comprises three tolerance compensation elements (11a, 11b, 11c), tolerance compensation elements (11a, 11b, 11c) being arranged on the distal end (13) of the collar element (7) such that two central axes (15a, 15b, 15c) of the tolerance compensating elements (11a, 11b, 11c) enclose an angle (α, β, γ) in each case.

8. Safety device (1) according to any of the preceding claims,
**characterised in that**
the at least one tolerance compensation element (11a, 11b, 11c) is integral with the collar element (7).

9. Safety device (1) according to any of the preceding claims,
**characterised in that**
the collar element (7) comprises a proximal region (20) that has an internal taper (21).

10. Safety device (1) according to claim 9,
**characterised in that**
at least one protruding element (22a, 22b, 22c) is arranged at least in the proximal region (20) of the collar element (7), the protruding element (22a, 22b, 22c) extending inwards in the radial direction (R) from the collar element (7).

11. Safety device (1) according to any of the preceding claims,
**characterised in that**
the safety device (1) comprises at least one spring element (23) that is operatively connected to the syringe body (3) and counteracts the movement of the sleeve element (6) relative to the safety device (1).

12. Safety device (1) according to any of the preceding claims,
**characterised in that**
the collar element (7) and/or the at least one tolerance compensation element (11a, 11b, 11c) consist of slip-modified polyoxymethylene (POM) or of a silicone compound.

13. Safety device (1) according to any of the preceding claims,
**characterised in that**
the collar element (7) and/or the at least one tolerance compensation element (11a, 11b, 11c) comprise a slip-promoting coating.

## Revendications

1. Dispositif de sécurité (1) pour éviter des blessures par piqûre pour une seringue (2) comportant un corps de seringue (3) et un moyen de perçage (5) disposé sur l'extrémité distale (4) du corps de seringue (3), comportant un élément manchon (6) s'étendant le long d'une direction axiale (X), lequel enferme au moins partiellement le moyen de perçage (5) et le corps de seringue (3), et un élément collier (7), lequel est disposé sur une zone d'extrémité distale (8) du corps de seringue (3) et verrouille le dispositif de sécurité (1) en direction axiale (X), l'élément collier (7) présentant au moins une saillie de guidage (9), laquelle est guidée dans au moins une coulisse de guidage (10) de l'élément manchon (6) sensiblement le long de la direction axiale (X) lors d'un mouvement relatif du corps de seringue (3) par rapport à l'élément manchon (6),
dans lequel
l'élément collier (7) est disposé apte à tourner dans une direction périphérique (U) sur la zone d'extrémité distale (8) du corps de seringue (3) et présente au moins un élément (11a, 11b, 11c) de compensation des tolérances, lequel est en contact fonctionnel, mécaniquement, avec le corps de seringue (3), de telle sorte que des tolérances du corps de seringue (3) soient compensables, le mouvement relatif du corps de seringue (3) par rapport à l'élément manchon (6) provoquant la rotation de l'élément collier (7) dans la direction périphérique (U) par le guidage de la saillie de guidage (9) dans ladite au moins une coulisse de guidage (10) de l'élément manchon (6),
**caractérisé par le fait que**
l'élément collier (7) comporte une zone distale (18) dans laquelle la paroi de l'élément collier (7) présente au moins deux fentes (19a, 19b), lesquelles s'étendent en direction axiale (X).

2. Dispositif de sécurité (1) selon la revendication 1,
**caractérisé par le fait que**
l'élément collier (7) est réalisé sensiblement en tant que cylindre à base circulaire creux (12), le cylindre à base circulaire (12) présentant une surface d'enveloppe (12a), sur laquelle ladite au moins une saillie de guidage (9) est disposée.

3. Dispositif de sécurité (1) selon l'une des revendications 1 ou 2,
**caractérisé par le fait que**
ledit au moins un élément (11a, 11b, 11c) de compensation des tolérances fait saillie au-delà d'une extrémité distale (13) de l'élément collier (7) le long de la direction axiale (X).

4. Dispositif de sécurité (1) selon l'une des revendications précédentes, **caractérisé par le fait que**
ledit au moins un élément (11a, 11b, 11c) de compensation des tolérances s'étend vers l'intérieur le long d'une direction radiale (R) à partir de l'élément collier (7).

5. Dispositif de sécurité (1) selon l'une des revendications précédentes, **caractérisé par le fait que**
ledit au moins un élément (11a, 11b, 11c) de compensation des tolérances est formé comme poinçon, ledit au moins un élément (11a, 11b, 11c) de compensation des tolérances en forme de poinçon présentant une surface de contact (12a, 21b, 12c), laquelle est apte à être mise en contact fonctionnel avec le corps de seringue (3).

6. Dispositif de sécurité (1) selon l'une des revendications précédentes, **caractérisé par le fait que**
ledit au moins un élément (11a, 11b, 11c) de compensation des tolérances est conçu élastique.

7. Dispositif de sécurité (1) selon l'une des revendications précédentes, **caractérisé par le fait que**
l'élément collier (7) comporte trois éléments (11a, 11b, 11c) de compensation des tolérances, les éléments (11a, 11b, 11c) de compensation des tolérances étant disposés sur l'extrémité distale (13) de l'élément collier (7) de telle sorte qu'à chaque fois deux axes médians (15a, 15b, 15c) des éléments (11a, 11b, 11c) de compensation des tolérances forment un angle (α, β, γ).

8. Dispositif de sécurité (1) selon l'une des revendications précédentes, **caractérisé par le fait que**
ledit au moins un élément (11a, 11b, 11c) de compensation des tolérances est formé intégrant avec l'élément collier (7).

9. Dispositif de sécurité (1) selon l'une des revendications précédentes, **caractérisé par le fait que**
l'élément collier (7) comporte une zone proximale (20), laquelle présente un cône intérieur (21).

10. Dispositif de sécurité (1) selon la revendication 9,
**caractérisé par le fait que**
au moins un élément faisant saillie (22a, 22b, 22c) est disposé au moins dans la zone proximale (20) de l'élément collier (7), l'élément faisant saillie (22a, 22b, 22c) s'étendant vers l'intérieur en direction radiale (R) à partir de l'élément collier (7).

11. Dispositif de sécurité (1) selon l'une des revendications précédentes, **caractérisé par le fait que**
le dispositif de sécurité (1) présente au moins un élément ressort (23), qui est relié fonctionnellement au corps de seringue (3) et s'oppose au mouvement relatif de l'élément manchon (6) par rapport au dispositif de sécurité (1).

12. Dispositif de sécurité (1) selon l'une des revendications précédentes, **caractérisé par le fait que**
l'élément collier (7) et/ou ledit au moins un élément (11a, 11b, 11c) de compensation des tolérances sont constitués de polyoxyméthylène (POM) à module de glissement modifié ou d'un composé silicone.

13. Dispositif de sécurité (1) selon l'une des revendications précédentes, **caractérisé par le fait que**
l'élément collier (7) et/ou ledit au moins un élément (11a, 11b, 11c) de compensation des tolérances présentent un revêtement favorisant le glissement.
